Europäisches Patentamt

**European Patent Office**

Office européen des brevets

(19)

(11) Numéro de publication: **0 241 391**
**B1**

(12)
# FASCICULE DE BREVET EUROPEEN

(45) Date de publication du fascicule du brevet:
**01.08.90**

(51) Int. Cl.⁵: **B01J 23/66, C07D 301/10**

(21) Numéro de dépôt: **87420089.2**

(22) Date de dépôt: **02.04.87**

(54) **Catalyseurs à base d'argent pour la fabrication d'oxyde d'éthylène.**

(30) Priorité: **11.04.86 FR 8605410**

(43) Date de publication de la demande:
**14.10.87 Bulletin 87/42**

(45) Mention de la délivrance du brevet:
**01.08.90 Bulletin 90/31**

(84) Etats contractants désignés:
**AT BE DE ES FR GB IT NL**

(56) Documents cités:
**EP-A- 0 002 143**
**EP-A- 0 005 388**
**EP-A- 0 017 725**
**EP-A- 0 097 935**
**GB-A- 2 043 481**
**GB-A- 2 117 263**
**US-A- 4 368 144**

(73) Titulaire: **ATOCHEM, 4 & 8, Cours Michelet La Défense 10, F-92800 Puteaux(FR)**

(72) Inventeur: **Cognion, Jean-Marie, 85 Route de Charly, F-69230 St Genis Laval(FR)**
Inventeur: **Letray, Gérard, 60, rue du Docteur Postel, F-76620 Le Havre(FR)**

ACTORUM AG

## Description

La présente invention concerne des catalyseurs à l'argent destinés à la fabrication d'oxyde d'éthylène par oxydation de l'éthylène par l'oxygène moléculaire.

Elle concerne plus particulièrement des catalyseurs dans lesquels l'argent est déposé sur un support réfractaire poreux qui sont modifiés par la présence d'au moins un promoteur déposé sur ledit support choisi parmi le césium, le rubidium, le potassium, la préparation de tels catalyseurs et leur utilisation pour fabriquer l'oxyde d'éthylène.

Les supports mis en jeu sont le plus souvent formés d'alumine, de silice-alumine, de magnésie, de pierre-ponce, de zircone, d'argile, de céramique, de graphite naturel ou artificiel, d'amiante, de zéolithe naturelle ou articifielle ou encore de carbure de silicium, sous des formes diverses, extrudés, anneaux, billes, etc., de dimension extérieure le plus souvent comprise entre 2 mm et 10 mm.

Les supports préférés présentent une faible surface spécifique, généralement égale au plus à 10 m²/g, de préférence inférieure à 1 m²/g, et une porosité élevée qui en volume est souvent supérieure à 20 % et peut atteindre 60 %.

La surface spécifique est déterminée dans la majorité des cas par la méthode d'adsorption de l'azote ou méthode B.E.T. décrite par BRUNAUER, EMMET et TELLER dans "The Journal of American Chemical Society", 60, p. 309, 1938, tandis que la porosité est mesurée par la méthode du porosimètre à mercure préconisée par E.W. WASHBURN dans les "Proceedings of the National Academy of Sciences of USA", 7, p.115, 1927, et décrite par L.C. DARKE dans "Industrial and Engineering Chemistry", 41, p.780, 1949 et dans "Industrial and Engineering Chemistry, Analytical Edition", 17, p.782, 1945.

Parmi les supports possédant les caractéristiques citées plus haut, les plus utilisés sont du type alumine pratiquement pure ou à faible teneur en silice.

L'alumine-$\alpha$ est particulièrement appréciée.

Le choix de tels supports préférés ne suffit pas en lui-même car l'activité, la sélectivité en oxyde d'éthylène et la tenue dans le temps des catalyseurs dépendent non seulement de lui, mais aussi de la nature des précurseurs des constituants actifs et de la technique utilisée pour déposer l'argent et les promoteurs sur le support.

Il est connu que deux techniques sont utilisées à ce jour : l'enrobage du support à l'aide des précurseurs des phases actives en suspension dans un liquide, l'imprégnation du support par ces précurseurs sous forme solubilisée.

A ce jour, il est communément admis que la seconde technique est plus favorable que la première, à cause en particulier d'une adhérence plus forte des constituants actifs.

L'art antérieur qualifie usuellement de "simultanée" l'imprégnation dans laquelle l'argent est toujours accompagné du promoteur lors de l'opération de dépôt, de "sequentielle" l'imprégnation dans laquelle le dépôt de la totalité de l'argent est complètement disjoint de celui de promoteur.

Comme le montrent par exemple les brevets des Etats-Unis d'Amérique n° 3 563 914, 3 962 136, 4 033 903, 4 010 115, 4 012 425 et 4 207 210 ou le brevet belge n° 793 658, l'imprégnation séquentielle équivaut pratiquement à l'imprégnation simultanée lorsque le dépôt du promoteur précède celui de l'argent mais est nettement inférieure à elle dans le cas contraire.

Selon la demande de brevet européen publiée sous le n° 0076504, même la transormation de l'argent déposé en argent métallique préalablement au dépôt du promoteur ne suffit pas à rendre l'imprégnation séquentielle supérieure à l'imprégnation simultanée.

Bien que l'industrie ait eu le souci permanent d'améliorer les catalyseurs, même de façon peu spectaculaire étant donné l'importance de l'enjeu économique dans le cadre d'un procédé industriel important pour la production à grande échelle d'oxyde d'éthylène, elle n'a pas dérogé au dépôt sur le support, par la technique d'imprégnation, de la totalité de l'argent et de la totalité du promoteur de façon soit entièrement simultanée, soit entièrement séparée.

Rien d'ailleurs ne l'y incitait puisque deux voies aussi éloignées l'une de l'autre que l'imprégnation simultanée l'est de l'imprégnation séquentielle, s'équivalaient finalement.

Il a été proposé de regrouper ces deux techniques pour les exécuter simultanément. Par exemple la demande de brevet européen publiée sous le n° 017 725 décrit un procédé dans lequel une suspension d'oxyde d'argent dans une solution aqueuse de sel organique d'argent est appliquée au support, le support ainsi traité est séché avant que lui soit appliqué à nouveau ladite suspension contenant cette fois les promoteurs sous forme d'un composé de baryum avec un composé de cesium et/ou de rubidium. Un tel procédé présente des rigidités dues à la nécessité qu'il y a à maintenir le rapport argent solide/argent solubilisé dans des limites fixées, et dues aux contraintes pratiques d'exécution des opérations de dépôt, de séchage et d'activation.

A ce jour, il est communément admis que l'imprégnation est plus favorable que l'enrobage, à cause en particulier d'une adhérence plus forte des constituants actifs.

Les catalyseurs de l'invention ont des performances sensiblement accrues par rapport à celles des catalyseurs connus. Ils sont obtenus selon le procédé de l'invention qui se distingue essentiellement des procédés connus faisant appel à l'imprégnation des supports par le fait que l'argent n'est déposé en totalité ni en même temps que le promoteur, ni séparément de lui.

Le procédé de fabrication de catalyseurs selon l'invention est caractérisé en ce qu'une partie de l'argent est déposée sur le support réfractaire poreux en imprégnant celui-ci par immersion dans une solution pratiquement anhydre d'un sel d'argent d'acide organique carboxylique dans un liquide organique complexant des ions argent, le liquide organique en excès est éliminé du support sans provoquer sur celui-ci le dépôt d'argent métallique, le complément d'argent et au moins un promoteur choisi parmi le césium, le rubidium, le potassium sont déposés sur le support modifié par le premier dépôt d'argent en imprégnant celui-ci par immersion dans une solution pratiquement anhydre d'un sel d'argent d'acide organique carboxylique dans un liquide organique complexant des ions argent renfermant à l'état solubilisé un sel d'au moins un des promoteurs, avant transformation de l'argent déposé en argent métallique.

La quantité d'argent dans le catalyseur peut varier largement, par exemple entre 5 % et 20 % en poids, de préférence entre 8 % et 16 %.

L'argent dans le catalyseur résulte de précurseurs, sels d'acides aliphatiques ou aromatiques comme par exemple l'acétate d'argent ou le benzoate d'argent, mis en œuvre directement ou formés au sein du liquide organique à partir d'oxyde d'argent et de l'acide.

De préférence, la quantité d'argent déposé lors de l'imprégnation du support modifé par le premier dépôt d'argent est le plus souvent comprise entre 25 % et 75 % de la quantité totale d'argent à déposer.

La quantité de promoteur déposé, exprimée en métal dans le catalyseur, peut aussi varier dans d'assez larges limites, le plus souvent entre 0,005 % et 0,05 % en poids, de préférence entre 0,01 % et 0,03 %.

Le promoteur peut être engagé directement sous forme de sel tel que de préférence l'acétate, le chlorure, le nitrate, ou sous forme d'oxyde transformé en sel dans le liquide organique par action de l'acide choisi.

Parmi les liquides organiques utilisés pour former les solutions d'imprégnation, sont préférés les hétérocycles contenant trois atomes de carbone, un atome d'oxygène et un atome d'azote préconisés par le brevet européen publié sous le numéro 0005388 et en particulier l'oxazole, la methyl-2-oxazoline-2 ou l'éthyl-2-oxazoline-2.

Parmi les supports préférés cités en début de texte conviennent indéniablement le mieux ceux qui, composés principalement ou pratiquement exclusivement d'alumine-$\alpha$ ont une surface spécifique comprise entre $0,1 \, m^2/g$ et $5 \, m^2/g$, de préférence inférieure à $1 \, m^2/g$, et une porosité en volume comprise entre environ 20 % et 50 %.

Avant chaque imprégnation le produit à imprégner est dégazé à une température de préférence comprise entre 50°C et 100°C sous une pression absolue n'excédant pas environ $25.10^{-3}$ bar, pendant une durée qui dans ces conditions est comprise entre 0,5 heure et 5 heures environ.

Les imprégnations peuvent être réalisées selon toute technique connue permettant une immersion complète du solide à imprégner, soit d'une façon statique, soit d'une façon dynamique par circulation de la solution d'imprégnation, à une température le plus souvent comprise entre 20°C et 60°C pendant environ 1/4 d'heure à 2 heures selon les cas.

Après la première imprégnation du support, le liquide organique en excès est éliminé de préférence par chauffage du support imprégné égoutté à une température au plus égale à 100°C sous courant d'azote de débit compris, par kilogramme, entre 5 l/heure et $0,5 \, m^3/heure$, la durée de l'opération ne réclamant alors généralement pas plus de 24 heures.

Une opération analogue à celle ci-dessus peut être effectuée, mais alors de façon facultative, après la seconde imprégnation.

La formation finale d'argent métallique est préférentiellement réalisée par traitement thermique du support modifié par les dépôts successifs d'argent d'une part, d'argent et de promoteur d'autre part.

Un traitement thermique particulièrement adopté consiste à porter le solide à traiter d'abord sous courant d'azote de débit compris, par kilogramme, entre 5 l/heure et $0,5 \, m^3/heure$, à une température comprise entre environ 150°C et 300°C atteinte après une montée en température de vitesse préférentiellement égale au plus à 50°C/heure environ, durant au moins le temps nécessaire pour qu'il ne soit pratiquement plus constaté de perte de poids, puis à le refroidir sous le courant d'azote à une température au plus égale à environ 100°C, enfin à le porter sous courant d'air de débit compris, par kilogramme, entre 5 l/heure et $0,5 \, m^3/heure$, à une température comprise de préférence entre 150°C et 200°C atteinte après une montée en température de vitesse comprise au mieux entre 50°C/heure et 100°C/heure, durant au moins le temps nécessaire pour qu'il ne soit pratiquement plus constaté la formation de dioxyde de carbone.

Les catalyseurs de l'invention, caractérisés en ce qu'ils résultent du mode d'obtention défini plus haut, présentent l'avantage de conduire à des résultats améliorés par rapport à ceux atteints avec les catalyseurs connus résultant des procédés connus impliquant l'imprégnation, lorsqu'ils sont mis en œuvre dans un procédé de fabrication d'oxyde d'éthylène abondamment décrit comme par exemple dans la "Revue de l'Institut Français du Pétrole", XI, n° 4, pp. 490-500 ou plus récemment dans "Chemical Engineering Progress" 75, n° 1, 1979, pp. 67-72.

Les exemples suivants, donnés à titre indicatif mais non limitatif, illustrent l'invention et permettent d'en apprécier l'avantage.

EXEMPLE 1

Cet exemple illustre la préparation de catalyseurs, selon l'invention : A-1, A-2, A-3, A-4, A-5, B-1, et de catalyseurs fabriqués dans un but de comparaison selon un procédé connu d'imprégnation "simultanée" : catalyseurs AC-1 et AC-2.

Les pourcentages, dans cet exemple comme d'ailleurs dans les exemples suivants sont des pourcentages en poids.

50 g d'un support A constitué d'alumine-α pratiquement pure du type SAHT, de surface spécifique inférieure à 1 m²/g, fabriqué par la Société UNION CATALYSTS INC. sous forme d'anneaux extrudés de diamètre extérieur 6,4 mm, de diamètre intérieur 3,2 mm et de longueur 7 à 8 mm, sont dégazés durant 2 heures à 50°C à une pression absolue de 25.10⁻³ bar puis imprégnés par immersion totale à 40°C durant 30 minutes dans une solution d'acétate d'argent dans l'éthyl-2-oxazoline-2 titrant 24 % d'acétate d'argent.

Le support ainsi imprégné est traité, après égouttage, à une température de 90°C durant 18 heures sous courant d'azote de débit égal à 5 l/heure avant d'être dégazé à 80°C durant 2 heures à une pression absolue de 25.10⁻³ bar et d'être soumis ensuite à une imprégnation par complète immersion à 40°C durant 30 minutes dans une solution d'acétate d'argent dans l'éthyl-2-oxazoline-2 titrant 24 % d'acétate d'argent et renfermant une quantité d'acétate de césium nécessaire pour obtenir un catalyseur contenant 0,03 % de césium.

Après égouttage, un traitement à 90°C sous azote est appliqué qui est identique à celui faisant suite à la première imprégnation.

Le solide résultant est traité sous 7,5 l/heure d'azote durant 18 heures à une température de 270°C atteinte après une montée en température à la vitesse de 20°C/heure, puis est refroidi à 80°C avant d'être traité sous 7,5 l/heure d'air durant 48 heures à une température de 175°C atteinte en 1 heure.

Le catalyseur A-1 ainsi obtenu renferme 0,03 % de césium et 9 % d'argent dont 25 % ont été déposés avec le césium.

Selon la même technique opératoire que pour A-1 un catalyseur A-2 est préparé en procédant au premier dépôt d'argent par immersion du support A dans une solution d'acétate d'argent dans la pyridine titrant 35 % d'acétate d'argent et en procédant au dépôt du complément d'argent et du promoteur par immersion dans une solution d'acétate d'argent dans la pyridine titrant 35 % d'acétate d'argent et renfermant la quantité requise d'acétate de césium pour obtenir un catalyseur contenant 0,015 % de césium.

Le catalyseur A-2 renferme ainsi 14,5 % d'argent et 0,015 % de césium.

Toujours selon la technique adoptée pour A-1 mais avec une solution d'imprégnation renfermant le complément d'argent et le césium qui titre 40 % d'acétate d'argent, les catalyseurs A-3 et A-4 sont obtenus qui renferment tous deux 13 % d'argent et respectivement 0,01 % et 0,03 % de césium. Pour A-3 et pour A-4 le complément d'argent déposé avec le césium correspond à 60 % de la quantité totale de l'argent déposé.

Selon la même technique que pour les catalyseurs ci-dessous mais en effectuant le dépôt d'argent seul par immersion du support dans une solution de benzoate d'argent dans l'oxazole titrant 31 % de benzoate d'argent et en effectuant le complément d'argent, qui s'élève à 60 % de la totalité de l'argent finalement déposé, et du césium, par immersion du support modifié par le premier dépôt d'argent dans une solution de benzoate d'argent dans l'oxazole titrant 55 % de benzoate d'argent et renfermant la quantité d'acétate de césium nécessaire à l'obtention d'un catalyseur contenant 0,025 % de césium, un catalyseur A-5 est préparé qui contient avec 0.025 % de césium, 14,5 % d'argent.

Un support B qui ne diffère du support A que par sa longueur qui n'est plus que de 3 à 4 mm, sert pour fabriquer le catalyseur B-1 comme a été fabriqué le catalyseur A-4. B-1, comme A-4 contient 13 % d'argent et 0,03 % de césium.

Le catalyseur AC-1 a été préparé suivant une technique connue en procédant après dégazage du support A à une imprégnation "simultanée" de celui-ci par immersion dans une solution d'acétate d'argent dans l'éthyl-2-oxazoline-2 titrant 32 % d'acétate d'argent et renfermant la quantité d'acétate de césium permettant d'obtenir un catalyseur contenant 0,032 % de césium.

Après élimination de l'éthyl-2-oxazoline-2 en excès comme décrit dans le cas des autres catalyseurs, le support, sur lequel a ainsi été déposé simultanément la totalité de l'argent et la totalité du césium, est traité pour transformer l'argent déposé en argent métallique comme décrit pour les catalyseurs de l'invention.

Le catalyseur AC-1 contient 9,2 % d'argent ce qui permet sa comparaison avec A-1.

Le catalyseur AC-2 a été préparé, en engageant le support A, selon la technique adoptée pour les catalyseurs de l'invention à la différence essentielle près que le promoteur a accompagné l'argent aussi bien lors de la première que de la seconde imprégnation, chacune réalisée par immersion dans une solution d'acétate d'argent dans la pyridine titrant 35 % d'acétate d'argent et contenant les deux fois la même quantité d'acétate de césium. Le catalyseur AC-2 renferme 14,3 % d'argent et 0,015 % de césium et peut donc être comparé à A-2.

## EXEMPLE 2

Cet exemple illustre l'utilisation des catalyseurs de l'invention et, à titre de comparaison, des catalyseurs connus, pour fabriquer l'oxyde d'éthylène.

Dans tous les essais reportés dans cet exemple, 40 g de catalyseur sont disposés dans un réacteur en verre de 25 mm de diamètre chauffé par circulation externe d'air chaud, au-dessus d'un lit de billes de verre servant à achever le préchauffage du mélange gazeux des réactifs qui entre par le bas du réacteur au débit de 20 l/heure mesuré à 20°C et à la pression atmosphérique à laquelle sont réalisés les essais.

Le mélange des réactifs a la composition volumique suivante :

éthylène : 17,5 %
oxygène : 7,0 %
éthane : 1,0 %
dioxyde de carbone : 6,0 %
chlorure de vinyle monomère : $1,5.10^{-4}$ %

Le complément à 100 % est assuré par l'azote.

La sélectivité de la réaction en oxyde d'éthylène, désignée par S, est exprimée, en %, par le nombre de moles d'oxyde d'éthylène formé par rapport au nombre de moles d'éthylène transformé.

Les résultats des essais effectués sont portés dans le tableau I ci-après qui indique aussi, pour chaque valeur de S la charge C définie comme la concentration d'oxyde d'éthylène, en % en volume, dans le mélange gazeux sortant du réacteur.

### TABLEAU I

| Catalyseur | Température °C | C | S |
|---|---|---|---|
| A-1 | 180 | 1 | 82,0 |
|  | 205 | 1,5 | 82 |
| AC-1 | 205 | traces |  |
|  | 230 | 1 | 82 |
| A - 2 | 183 | 1 | 82 |
| AC-2 | 183 | 1 | 80,5 |
| A - 3 | 190 | 1 | 80,5 |
| A - 4 | 180 | 1 | 82 |
|  | 194 | 1,8 | 80,5 |
| A - 5 | 186 | 1 | 81 |
|  | 191 | 1,8 | 80 |
| B-1 | 200 | 1 | 85 |
|  | 212 | 1,5 | 84 |

## EXEMPLE 3

46,5 g de catalyseur B-1 sont disposés dans le réacteur de l'exemple 2 et essayés dans les conditions suivantes : le mélange gazeux des réactifs entre dans le réacteur au débit de 25 l/heure mesuré comme dans l'exemple 2 et a la même composition volumique que dans cet exemple, à l'exception du complément à 100 % qui est assuré par le méthane à la place de l'azote.

Les résultats obtenus sont rassemblés dans le tableau II, C et S ayant la même signification que dans l'exemple 2.

## TABLEAU II

| Température °C | C | S |
|---|---|---|
| 196 | 0,8 | 84 |
| 199 | 1,6 | 82 |

Il ressort des tableaux I et II que les catalyseurs préparés selon le procédé de l'invention constituent une amélioration sensible par rapport au domaine connu et permettent en particulier le maintien d'une sélectivité élevée quand la concentration en oxyde d'éthylène à la sortie du réacteur est elle-même élevée, et une grande sureté de fonctionnement, les qualités ci-dessus se concrétisant à basse température.

Le remplacement, pour la préparation des catalyseurs selon l'invention, de l'oxazole ou de l'éthyl-2-oxazoline-2 par un composé différent mais de même nature comme la méthyl-2-oxazoline-2, n'a pas d'incidence appréciable.

L'effet d'amélioration dû à l'invention subsiste que le césium soit engagé seul ou substitué en partie ou en totalité par le potassium et/ou le rubidium.

**Revendications**

1. Procédé de fabrication de catalysateurs à l'argent déposé sur un support réfractaire poreux, destinés à la fabrication d'oxyde d'éthylène par oxydation de l'éthylène par l'oxygène moléculaire, modifiés par la présence d'au moins un promoteur déposé sur ce support, caractérisé en ce qu'une partie de l'argent est déposée sur le support en imprégnant celui-ci par immersion dans une solution d'un sel d'argent d'acide organique carboxylique dans un liquide organique complexant des ions argent, le liquide organique en excès est éliminé du support sans provoquer sur celui-ci le dépôt d'argent métallique, le complément d'argent et au moins un promoteur, choisi parmi le césium, le rubidium, le potassium, sont déposés sur le support modifié par le premier dépôt d'argent, en l'imprégnant par immersion dans une solution d'un sel d'argent d'acide organique carboxylique dans un liquide organique complexant des ions argent renfermant à l'état solubilisé un sel d'au moins un des promoteurs avant transformation de l'argent déposé en argent métallique.

2. Procédé selon la revendication 1, caractérisé en ce que le complément d'argent déposé est compris entre 25% et 75% de la quantité totale d'argent déposé.

3. Procédé selon l'une des revendications 1 et 2, caractérisé en ce que la quantité totale d'argent déposé est comprise entre 5% et 20% en poids du catalysateur.

4. Procédé selon la revendication 3, caractérisé en ce que la quantité totale d'argent déposé est comprise entre 8% et 16% en poids du catalysateur.

5. Procédé selon l'une des revendications 1 à 4, caractérisé en ce que le sel d'argent est choisi parmi l'acétate d'argent et le benzoate d'argent.

6. Procédé selon l'une des revendications 1 à 5, caractérisé en ce que la quantité de promoteur déposé, comptée en métal, est comprise entre 0,005% et 0,05% en poids du catalysateur.

7. Procédé selon la revendication 6, caractérisé en ce que la quantité de promoteur déposé est comprise entre 0,01% et 0,03% en poids du catalysateur.

8. Procédé selon l'une des revendications 1 à 7, caractérisé en ce que le sel du promoteur est choisi parmi l'acétate, le chlorure, le nitrate.

9. Procédé selon l'une des revendications 1 à 8, caractérisé en ce que le liquide organique complexant des ions argent est choisi parmi la pyridine, l'oxazole, la méthyl-2-oxazoline-2, l'éthyl-2-oxazoline-2.

10. Procédé selon l'une des revendications 1 à 9, caractérisé en ce que l'excès de liquide organique est éliminé du support imprégné par chauffage de celui-ci à une température au plus égale à 100°C sous courant d'azote.

11. Procédé selon la revendication 10, caractérisé en ce qu'après élimination du liquide organique en excès le support modifié par le premier dépôt d'argent est dégazé avant dépôt du complément d'argent et du promoteur, à une pression absolue au plus égale à $25.10^{-3}$ bar et à une température comprise entre 50°C et 100°C.

12. Procédé selon l'une revendications 1 à 11, caractérisé en ce que la formation d'argent métallique est réalisée par le traitement thermique du support modifié par les dépôts successifs d'argent d'une part, d'argent et de promoteur d'autre part, d'abord sous courant d'azote à une température comprise entre 150°C et 300°C durant au moins le temps nécessaire pour qu'il ne soit pratiquement plus constaté de perte de poids, ensuite sous courant d'air à une température comprise entre 150°C et 200°C durant au moins le temps nécessaire pour qu'il ne soit pratiquement plus constaté la formation de dioxyde de carbone.

13. Procédé selon l'une des revendications 1 à 12, caractérisé en ce que le support est une alumine-α pratiquement pure de surface spécifique inférieure à 1 m²/g.

14. Catalyseurs à l'argent déposé sur support poreux réfractaire modifiés par la présence d'au moins un promoteur, choisi parmi le césium, le rubidium, le potassium, caractérisé en ce qu'ils sont fabriqués selon un procédé caractérisé comme dans l'une quelconque des revendications 1 à 13.

## Claims

1. A process for manufacturing catalysts of silver deposited on a porous refractory support, the catalysts being adapted to the manufacture of ethylene oxide by the oxidation of ethylene with molecular oxygen and modified by the presence of at least one promoter deposited on the support, characterized in that a portion of the silver is deposited on the support by impregnating the support by immersion in a solution of a silver salt of organic carboxylic acid in an organic liquid complexing silver ions, the surplus organic liquid is eliminated from the support without causing the deposit of metallic silver thereon, the complement of silver and at least one promoter selected from caesium, rubidium and potassium are deposited on the support modified by the first silver deposit by impregnating it by immersion in a solution of a silver salt of organic carboxylic acid in an organic liquid complexing silver ions containing in the solubilized state a salt of at least one of the promoters, prior to the conversion of the deposited silver into metallic silver.

2. A process according to claim 1, characterized in that the complement of silver deposited is between 25 and 75% of the total quantity of silver deposited.

3. A process according to one of claims 1 or 2, characterized in that the total quantity of silver deposited is between 5 and 20% by weight of the catalyst.

4. A process according to claim 3, characterized in that the total quantity of silver deposited is between 8 and 16% by weight of the catalyst.

5. A process according to one of claims 1 to 4, characterized in that the silver salt is selected from silver acetate and silver benzoate.

6. A process according to one of claims 1 to 5, characterized in that the quantity of promoter deposited, counted in metal, is between 0.005 and 0.05% by weight of the catalyst.

7. A process according to claim 6, characterized in that the quantity of promoter deposited is between 0.01 and 0.03% by weight of the catalyst.

8. A process according to one of claims 1 to 7, characterized in that the salt of the promoter is selected from the acetate, chloride and nitrate.

9. A process according to one of claims 1 to 8, characterized in that the organic liquid complexing silver ions is selected from pyridine, oxazole, 2-methyl-2-oxazoline and 2-ethyl-2-oxazoline.

10. A process according to one of claims 1 to 9, characterized in that the surplus of organic liquid is eliminated from the impregnated support by heating the latter to a temperature of at most 100°C in a flow of nitrogen.

11. A process according to claim 10, characterized in that after the elimination of the surplus organic liquid the support modified by the first silver deposit is degassed before the deposit of the complement of silver and the promoter at an absolute pressure of at most $25.10^{-3}$ bar and a temperature of between 50 and 100°C.

12. A process according to one of claims 1 to 11, characterized in that the formation of metallic silver is performed by thermal treatment of the support modified by the successive deposits of silver on the one hand and of silver and promoter on the other, at first in a flow of nitrogen at a temperature of between 150 and 300°C for at least the time necessary for practically no further weight loss to be observed, and thereafter in an airflow at a temperature of between 150 and 200°C for at least the time necessary for practically no further formation of carbon dioxide to be observed.

13. A process according to one of claims 1 to 12, characterized in that the support is a practically pure α alumina of specific surface less than 1 m²/g.

14. Silver catalysts deposited on a porous refractory support and modified by the presence of at least one promoter selected from caesium, rubidium and potassium, characterized in that the catalysts are manufactured by a process characterized as in any one of claims 1 to 13.

## Patentansprüche

1. Verfahren zur Herstellung von Katalysatoren auf Basis von Silber, das auf einen feuerfesten porösen Träger aufgebracht ist, bestimmt für die Herstellung von Ethylenoxid durch Oxydation von Ethylen mit molekularem Sauerstoff, modifiziert durch Anwesenheit wenigstens eines auf diesen Träger aufgebrachten Beschleunigers, dadurch gekennzeichnet, daß ein Teil des Silbers auf diesen Träger aufgebracht wird, indem man diesen durch Eintauchen in eine Lösung eines Silbersalzes einer organischen Carbonsäure in einer organischen Silberionen komplexierenden Flüssigkeit tränkt, wobei die überschüssige organische Flüssigkeit von dem Träger entfernt wird, ohne auf diesem die Ablagerung von metallischem Silber hervorzurufen, wobei das restliche Silber und wenigstens ein Beschleuniger, ausgewählt aus Cäsium, Rubidium und Kalium auf den durch die erste Ablagerung des Silbers modifizierten Träger aufgebracht werden, indem dieser imprägniert wird durch Eintauchen in eine Lösung eines Silbersalzes einer organischen Carbonsäure in einer organischen Silberionen komplexierenden Flüssigkeit, die in gelöstem Zustand ein Salz wenigstens eines der Beschleuniger enthält, bevor die Umwandlung des abgelagerten Silbers in metallisches Silber erfolgt.

2. Verfahren nach Anspruch 1, dadurch gekennzeichnet, daß das restliche aufgebrachte Silber zwischen 25 % und 75 % der Gesamtmenge des aufgebrachten Silbers beträgt.

3. Verfahren nach einem der Ansprüche 1 und 2, dadurch gekennzeichnet, daß die Gesamtmenge des aufgebrachten Silbers zwischen 5 % und 20 Gew.-% des Katalysators beträgt.

4. Verfahren nach Anspruch 3, dadurch gekennzeichnet, daß die Gesamtmenge des aufgebrachten Silbers zwischen 8 % und 16 Gew.-% des Katalysators beträgt.

5. Verfahren nach einem der Ansprüche 1 bis 4, dadurch gekennzeichnet, daß das Silbersalz ausgewählt wird aus Silberacetat und Silberbenzoat.

6. Verfahren nach einem der Ansprüche 1 bis 5, dadurch gekennzeichnet, daß die Menge des aufgebrachten Beschleunigers, berechnet als Metall zwischen 0,005 % und 0,05 Gew.-% des Katalysators ausmacht.

7. Verfahren nach Anspruch 6, dadurch gekennzeichnet, daß die Menge des aufgebrachten Beschleunigers zwischen 0,01 % und 0,03 Gew.-% des Katalysators beträgt.

8. Verfahren nach einem der Ansprüche 1 bis 7, dadurch gekennzeichnet, daß das Salz des Beschleunigers ausgewählt wird aus Acetat, Chlorid und Nitrat.

9. Verfahren nach einem der Ansprüche 1 bis 8, dadurch gekennzeichnet, daß die Silberionen komplexierende organische Flüssigkeit ausgewählt wird aus Pyridin, Oxazol, 2-Methyl-3-oxazolin, 2-Ethyl-2-oxazolin.

10. Verfahren nach einem der Ansprüche 1 bis 9, dadurch gekennzeichnet, daß der Überschuß an organischer Flüssigkeit vom imprägnierten Träger durch Erwärmung des Trägers auf eine Temperatur von höchstens 100°C unter Stickstoffstrom entfernt wird.

11. Verfahren nach Anspruch 10, dadurch gekennzeichnet, daß nach Entfernung der überschüssigen organischen Flüssigkeit der durch die erste Aufbringung von Silber modifizierte Träger entgast wird, bevor die vollständige Aufbringung des Silbers und des Beschleunigers erfolgt, bei einem absoluten Druck von höchstens $25.10^{-3}$ bar und einer Temperatur zwischen 50°C und 100°C.

12. Verfahren nach einem der Ansprüche 1 bis 11, dadurch gekennzeichnet, daß die Bildung des metallischen Silbers durch thermische Behandlung des durch die aufeinanderfolgende Aufbringung des Silbers und des Silbers und des Beschleunigers modifizierten Trägers durchgeführt wird, zunächst unter Stickstoffstrom bei einer Temperatur zwischen 150°C und 300°C für eine solche Zeitdauer, die notwendig ist, daß praktisch kein Gewichtsverlust mehr feststellbar ist, und anschließend unter einem Luftstrom bei einer Temperatur zwischen 150°C und 200°C für wenigstens die Zeitdauer, die notwendig ist, bis praktisch keine Kohlendioxidbildung mehr feststellbar ist.

13. Verfahren nach einem der Ansprüche 1 bis 12, dadurch gekennzeichnet, daß der Träger ein praktisch reines α-Aluminiumoxid ist mit einer spezifischen Oberfläche unterhalb von 1 $m^2$/g.

14. Katalysatoren auf Basis von Silber aufgebracht auf einen feuerfesten porösen Träger modifiziert durch die Anwesenheit wenigstens eines Beschleunigers ausgewählt aus Cäsium, Rubidium und Kalium, dadurch gekennzeichnet, daß sie in einem Verfahren gemäß einem der Ansprüche 1 bis 13 hergestellt werden.